# EUROPEAN PATENT APPLICATION

(11) **EP 4 227 395 A1**
(43) Date of publication of application: **16.08.2023**
(21) Application number: 21877702.7
(22) Date of filing: 07.10.2021
(51) Int. Cl.: C12M 1/00, C12M 1/34, C12N 15/09, C12N 15/12, C12Q 1/6844, C12Q 1/686, C12Q 1/6888

(54) **ONE OR MORE HLA GENE PRIMERS**

(30) Priority: 08.10.2020 JP 2020170696
(71) Applicant: National Center for Global Health and Medicine, Tokyo 162-8655 (JP); H.U. Group Research Institute G.K., Akiruno-shi, Tokyo 197-0833 (JP)
(72) Inventor: TOKUNAGA, Katsushi, Tokyo 162-8655 (JP); KHOR, Seik-Soon, Tokyo 162-8655 (JP); OMAE, Yosuke, Tokyo 162-8655 (JP); NAITO, Ikue, Fuchigami, Tokyo 197-0833 (JP); SATO, Tetsuya, Fuchigami, Tokyo 197-0833 (JP); WAKABAYASHI, Shunichi, Fuchigami, Tokyo 197-0833 (JP)
(74) Representative: Müller-Boré & Partner Patentanwälte PartG mbB
(86) International application number: PCT/JP2021/037121
(87) International publication number: WO 2022/075403

(57) **Abstract**

The present invention provides one or more kinds of primers capable of highly comprehensively detecting or amplifying an HLA allele of an HLA-E gene, an HLA-F gene, or an HLA-G gene. More specifically, the present invention provides one or more kinds of HLA gene primers selected from the group consisting of the following (1) to (3):
(1) one or more kinds of HLA-G gene primers
(1a) a first primer comprising, for example, the nucleotide sequence of SEQ ID NO. 1 and/or (1b) a second primer comprising, for example, the nucleotide sequence of SEQ ID NO. 3;
(2) one or more kinds of HLA-E gene primers
(2a) a first primer comprising, for example, the nucleotide sequence of SEQ ID NO. 5 and/or (2b) a second primer comprising, for example, the nucleotide sequence of SEQ ID NO. 7; and
(3) one or more kinds of HLA-F gene primers
(3a) a first primer comprising, for example, the nucleotide sequence of SEQ ID NO. 9 and/or (3b) a second primer comprising, for example, the nucleotide sequence of SEQ ID NO. 11.

## Description

### FIELD

The present invention relates to one or more kinds of HLA gene primers and the like.

### BACKGROUND

A human leukocyte antigen (HLA) is an important molecule that controls immune responsiveness and is associated with various disease sensitivities. The HLA is roughly classified into a class I molecule and a class II molecule. The HLA class I molecule functions as a ligand of an immune receptor on a natural killer cell, a T cell, and a myeloid cell in addition to having a function of presenting an antigen peptide to a T cell.

The HLA class I molecule includes two groups of a classical (class Ia) molecule and a non-classical (class Ib) molecule. HLA-A, HLA-B, and HLA-C which are the HLA class Ia molecules are expressed in almost all human cells. Meanwhile, in HLA-E, HLA-F, and HLA-G which are the HLA class Ib molecules, a distribution of expressed tissues is often limited, and functions thereof are not limited to presentation of an antigen peptide but are diverse. For example, the HLA-G molecule is mainly expressed in the placenta and presents an antigen peptide like the HLA class Ia molecule. A receptor to which the antigen peptide is presented is a receptor of a natural killer cell, and the HLA-G molecule regulates a function of the natural killer cell. The natural killer cell is crucial in cancer immunotherapy.

The HLA class I gene groups have high sequence homology with each other, and are longitudinally arranged on the short arm of chromosome 6. Very high polymorphism is observed in the HLA class Ia gene, whereas polymorphism in the HLA class Ib gene is relatively low. Meanwhile, since the entire HLA gene region is highly rich in polymorphism, an intergenic region is also rich in polymorphism as compared with a general genomic region. Therefore, it is difficult to design a PCR primer capable of comprehensively amplifying the entire length of a gene of an HLA allele only with a human genome reference sequence (genome assembly version hg 38).

The HLA allele is defined by a combination of polymorphisms of the entire length of a gene. A sanger type sequencer or a next generation sequencer cannot sequence the entire length of a gene at a time because a sequence length that can be determined is short. In particular, since polymorphism in the HLA class Ib gene is relatively low, a probability that a polymorphic site is present in a determined sequence is low. Therefore, it is not possible to phase (phasing) HLA alleles derived from the mother and the father (Non Patent Literature 1). In addition, for the HLA class Ib gene, since there is no established HLA allele typing method, and no kit is commercially available, there are few cases of reports on typing results. Therefore, HLA class Ib gene HLA allele information registered in IMGT/HLA database is limited.

Nilsson et al. have reported one type of primer set capable of amplifying the entire length of the HLA-G gene including an untranslated region (Non Patent Literature 2).

Alizadeh et al. have reported a long-range PCR primer set targeting the HLA-F gene, but design the primer in an untranslated region (Non Patent Literature 3).

Wang et al. have reported a long-range PCR primer set targeting the HLA-E gene, but cannot amplify the entire length of the HLA-E gene including an untranslated region (Non Patent Literature 4). Lucas et al. have also reported a sequence result of the entire length of the HLA-E gene using a long-range PCR, but designs a primer in an untranslated region, and cannot amplify a region including an untranslated region that affects gene expression (Non Patent Literature 5).

### CITATION LIST

### NON PATENT LITERATURE

Non Patent Literature 1: Suzuki S et al., Front. Immunol. 2018 Oct 4;9:2294.
Non Patent Literature 2: Nilsson LL et al., HLA. 2018;92:144-153.
Non Patent Literature 3: Alizadeh M et al., Hum Immunol. 2020;81(5):202-205.
Non Patent Literature 4: Wang S-X et al., HLA. 2017;89:327-330.
Non Patent Literature 5: Lucas JAM et al., HLA. 2020;95:561-572.

### SUMMARY

### TECHNICAL PROBLEM

For the HLA-E gene and the HLA-F gene, a primer capable of amplifying the entire length of the gene including an untranslated region has not been reported.

For the HLA-G gene primer disclosed in prior art, as described in Examples, as a result of examination by analysis using whole genome sequence data, it has been confirmed that there is a mismatch in a primer binding site depending on a sample, and it has been suggested that PCR amplification is not observed or there is a high possibility that uniform amplification is affected.

In addition, in prior art, an HLA class Ib gene sequence is analyzed by PCR using a pair of primers designed around a translated region of the HLA class Ib gene, but by such a method, polymorphism in an untranslated region which is important for gene expression cannot be analyzed.

Therefore, an object of the present invention is to provide one or more kinds of primers capable of highly comprehensively detecting or amplifying an HLA allele of an HLA-E gene, an HLA-F gene, or an HLA-G gene. Another object of the present invention is to provide one or more kinds of primers capable of highly comprehensively detecting or amplifying the HLA allele of the above gene and capable of amplifying the entire length of the gene including an untranslated region of the gene.

### SOLUTION TO PROBLEM

As a result of intensive studies, the present inventors have found one or more kinds of HLA gene primers capable of highly comprehensively detecting or amplifying an HLA allele of an HLA-E gene, an HLA-F gene, or an HLA-G gene, and capable of amplifying the entire length of the gene including an untranslated region of the gene. Actually, the one or more kinds of HLA gene primers found by the present inventors have been actually confirmed to be able to highly comprehensively amplify a large number of HLA allele groups including a putative novel HLA allele, unlike prior art (Table 6). Based on such findings, the present inventors have succeeded in providing one or more kinds of HLA gene primers, thereby completing the present invention.

That is, the present invention is as follows.
[1] One or more kinds of HLA gene primers selected from the group consisting of the following (1) to (3):
   (1) one or more kinds of HLA-G gene primers which are
      (1a) a first primer comprising the nucleotide sequence of SEQ ID NO. 1 or the nucleotide sequence complementary thereto and/or (1b) a second primer comprising the nucleotide sequence of SEQ ID NO. 3 or the nucleotide sequence complementary thereto;
   (2) one or more kinds of HLA-E gene primers which are
      (2a) a first primer comprising the nucleotide sequence of SEQ ID NO. 5 or the nucleotide sequence complementary thereto and/or (2b) a second primer comprising the nucleotide sequence of SEQ ID NO. 7 or the nucleotide sequence complementary thereto; and
   (3) one or more kinds of HLA-F gene primers which are
      (3a) a first primer comprising the nucleotide sequence of SEQ ID NO. 9 or the nucleotide sequence complementary thereto and/or (3b) a second primer comprising the nucleotide sequence of SEQ ID NO. 11 or the nucleotide sequence complementary thereto.
[2] The one or more kinds of HLA gene primers according to [1], wherein the one or more kinds of HLA gene primers are HLA gene amplification primer sets selected from the group consisting of the following (1') to (3'):
   (1') an HLA-G gene amplification primer set including
      (1a) a first primer comprising the nucleotide sequence of SEQ ID NO. 1 or the nucleotide sequence complementary thereto and (1b) a second primer comprising the nucleotide sequence of SEQ ID NO. 3 or the nucleotide sequence complementary thereto;
   (2') an HLA-E gene amplification primer set including
      (2a) a first primer comprising the nucleotide sequence of SEQ ID NO. 5 or the nucleotide sequence complementary thereto and (2b) a second primer comprising the nucleotide sequence of SEQ ID NO. 7 or the nucleotide sequence complementary thereto; and
   (3') an HLA-F gene amplification primer set including
      (3a) a first primer comprising the nucleotide sequence of SEQ ID NO. 9 or the nucleotide sequence complementary thereto and (3b) a second primer comprising the nucleotide sequence of SEQ ID NO. 11 or the nucleotide sequence complementary thereto.
[3] The one or more kinds of HLA gene primers according to [1] or [2], wherein
   (1a) the first primer comprising the nucleotide sequence of SEQ ID NO. 1 or the nucleotide sequence complementary thereto is (1a') a first primer comprising the nucleotide sequence of SEQ ID NO. 2 or the nucleotide sequence complementary thereto,
   (1b) the second primer comprising the nucleotide sequence of SEQ ID NO. 3 or the nucleotide sequence complementary thereto is (1b') a second primer comprising the nucleotide sequence of SEQ ID NO. 4 or the nucleotide sequence complementary thereto,
   (2a) the first primer comprising the nucleotide sequence of SEQ ID NO. 5 or the nucleotide sequence complementary thereto is (2a') a first primer comprising the nucleotide sequence of SEQ ID NO. 6 or the nucleotide sequence complementary thereto,
   (2b) the second primer comprising the nucleotide sequence of SEQ ID NO. 7 or the nucleotide sequence complementary thereto is (2b') a second primer comprising the nucleotide sequence of SEQ ID NO. 8 or the nucleotide sequence complementary thereto,
   (3a) the first primer comprising the nucleotide sequence of SEQ ID NO. 9 or the nucleotide sequence complementary thereto is (3a') a first primer comprising the nucleotide sequence of SEQ ID NO. 10 or the nucleotide sequence complementary thereto, and
   (3b) the second primer comprising the nucleotide sequence of SEQ ID NO. 11 or the nucleotide sequence complementary thereto is (3b') a second primer comprising the nucleotide sequence of SEQ ID NO. 12 or the nucleotide sequence complementary thereto.
[4] A method for detecting an HLA gene, comprising:
   detecting one or more kinds of HLA genes in a sample obtained from a human subject using the one or more kinds of HLA gene primers according to any of [1] to [3],
   wherein the one or more kinds of HLA genes are selected from the group consisting of an HLA-G gene, an HLA-E gene, and an HLA-F gene.
[5] The method according to [4], wherein the one or more kinds of HLA genes are detected by amplifying the one or more kinds of HLA genes.
[6] An HLA gene detection reagent comprising the one or more kinds of HLA gene primers according to any of [1] to [3].
[7] An HLA gene detection kit comprising:
   (a) the one or more kinds of HLA gene primers according to any of [1] to [3]; and
   (b) a polymerase.

### EFFECTS OF INVENTION

According to the present invention, an HLA allele of an HLA-E gene, an HLA-F gene, or an HLA-G gene can be highly comprehensively detected or amplified. In addition, according to the present invention, the entire length of the HLA-E gene, the HLA-F gene, or the HLA-G gene including an untranslated region thereof can be amplified.

### BRIEF DESCRIPTION OF DRAWINGS

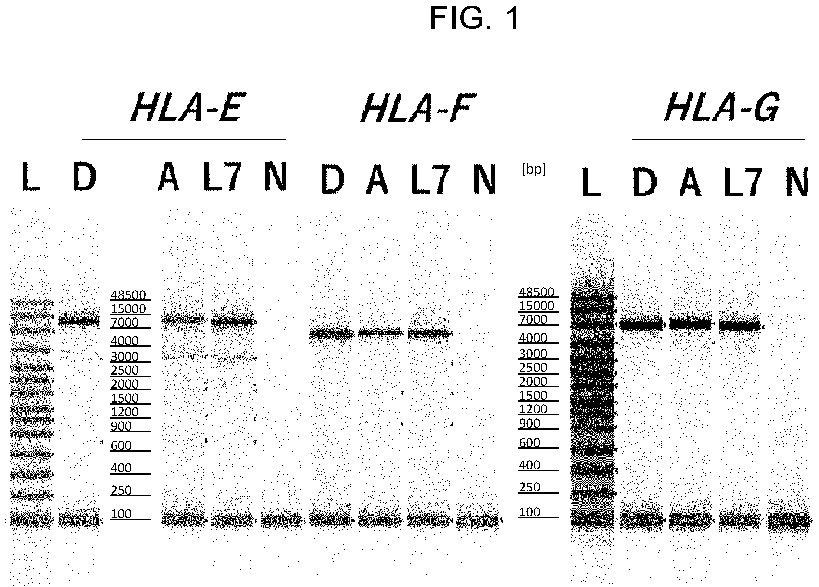

FIG. 1 is a diagram illustrating results of PCR amplification of an HLA-E gene, an HLA-F gene, and an HLA-G gene. L: molecular weight marker; D: Genomic DNA extracted from a cell line (Daudi) established from an African population was used as a template; A: Genomic DNA extracted from a cell line (AKIBA) established from a Japanese population was used as a template; L7: Genomic DNA extracted from Hispanic American-derived human peripheral blood mononuclear cells was used as a template; N: No genomic DNA was used (negative control).

### EMBODIMENTS FOR CARRYING OUT INVENTION

The present invention provides one or more kinds of HLA gene primers selected from the group consisting of the following (1) to (3):
(1) one or more kinds of HLA-G gene primers:
   (1a) a first primer comprising the nucleotide sequence of SEQ ID NO. 1 or the nucleotide sequence complementary thereto and/or (1b) a second primer comprising the nucleotide sequence of SEQ ID NO. 3 or the nucleotide sequence complementary thereto;
(2) one or more kinds of HLA-E gene primers:
   (2a) a first primer comprising the nucleotide sequence of SEQ ID NO. 5 or the nucleotide sequence complementary thereto and/or (2b) a second primer comprising the nucleotide sequence of SEQ ID NO. 7 or the nucleotide sequence complementary thereto; and
(3) one or more kinds of HLA-F gene primers:
   (3a) a first primer comprising the nucleotide sequence of SEQ ID NO. 9 or the nucleotide sequence complementary thereto and/or (3b) a second primer comprising the nucleotide sequence of SEQ ID NO. 11 or the nucleotide sequence complementary thereto.

The one or more kinds of primers of the present invention are not particularly limited as long as the primers can be annealed to a target site of an HLA gene selected from the group consisting of an HLA-G gene, an HLA-E gene, and an HLA-F gene. For example, as the one or more kinds of primers of the present invention, any primer containing a natural nucleic acid or an artificial nucleic acid can be used, but a DNA primer is preferable from a viewpoint of versatility, cost, and the like.

In an embodiment, the one or more kinds of primers of the present invention may be one HLA gene primer. For example, such one or more kinds of primers of the present invention are useful as sequencing primers or targets of an RNA-guided nuclease (for example, Cas9) because of having excellent HLA allele comprehensiveness.

When the one or more kinds of primers of the present invention are sequencing primers or targets of an RNA-guided nuclease, one primer is the following primer from a viewpoint of targeting a region in an HLA gene.
(1) one HLA-G gene primer:
   (1a) a first primer comprising the nucleotide sequence of SEQ ID NO. 1 or (1b) a second primer comprising the nucleotide sequence of SEQ ID NO. 3;
(2) one HLA-E gene primer:
   (2a) a first primer comprising the nucleotide sequence of SEQ ID NO. 5 or (2b) a second primer comprising the nucleotide sequence of SEQ ID NO. 7; or
(3) one HLA-F gene primer:
   (3a) a first primer comprising the nucleotide sequence of SEQ ID NO. 9 or (3b) a second primer comprising the nucleotide sequence of SEQ ID NO. 11.

When the one or more kinds of primers of the present invention are sequencing primers or targets of an RNA-guided nuclease, one primer may be (1a) a first primer comprising the nucleotide sequence of SEQ ID NO. 1, (2a) a first primer comprising the nucleotide sequence of SEQ ID NO. 5, or (3a) a first primer comprising the nucleotide sequence of SEQ ID NO. 9 from a viewpoint of targeting an untranslated region upstream of a gene that is particularly important for gene expression.

In another embodiment, the one or more kinds of primers of the present invention may be a primer set including two or more primers. For example, such a primer set is excellent in HLA allele comprehensiveness, and therefore is useful as a gene amplification primer set.

When the one or more kinds of primers of the present invention are a primer set including two or more primers, the one or more kinds of primers may be the following primer set including at least one specific primer excellent in HLA allele comprehensiveness.
(1) a primer set including two or more HLA-G gene primers:
   a primer set including (1a) a first primer comprising the nucleotide sequence of SEQ ID NO. 1 or the nucleotide sequence complementary thereto and (1b) a second primer comprising the nucleotide sequence of SEQ ID NO. 3 or the nucleotide sequence complementary thereto, or
   a primer set including a primer selected from (1a) a first primer comprising the nucleotide sequence of SEQ ID NO. 1 or the nucleotide sequence complementary thereto and (1b) a second primer comprising the nucleotide sequence of SEQ ID NO. 3 or the nucleotide sequence complementary thereto, and another primer capable of amplifying an HLA-G gene in combination with the selected primer;
(2) a primer set including two or more HLA-E gene primers:
   a primer set including (2a) a first primer comprising the nucleotide sequence of SEQ ID NO. 5 or the nucleotide sequence complementary thereto and (2b) a second primer comprising the nucleotide sequence of SEQ ID NO. 7 or the nucleotide sequence complementary thereto, or
   altenatively
   a primer set including a primer selected from (2a) a first primer comprising the nucleotide sequence of SEQ ID NO. 5 or the nucleotide sequence complementary thereto and (2b) a second primer comprising the nucleotide sequence of SEQ ID NO. 7 or the nucleotide sequence complementary thereto, and another primer capable of amplifying an HLA-E gene in combination with the selected primer; or
(3) two or more HLA-F gene primers:
   a primer set including (3a) a first primer comprising the nucleotide sequence of SEQ ID NO. 9 or the nucleotide sequence complementary thereto and (3b) a second primer comprising the nucleotide sequence of SEQ ID NO. 11 or the nucleotide sequence complementary thereto, or
   a primer set including a primer selected from (3a) a first primer comprising the nucleotide sequence of SEQ ID NO. 9 or the nucleotide sequence complementary thereto and (3b) a second primer comprising the nucleotide sequence of SEQ ID NO. 11 or the nucleotide sequence complementary thereto, and another primer capable of amplifying an HLA-F gene in combination with the selected primer.

The number of primers required for gene amplification varies depending on the type of gene amplification method. For example, in PCR, the number of primers required for gene amplification is two. Meanwhile, in loop-mediated isothermal amplification (LAMP) (see, for example, WO 00/28082 A), the number of primers required for gene amplification is 4 or 6. Therefore, when amplification by a gene amplification method requiring more than two primers is intended, the primer set may include an additional primer.

Preferably, the one or more kinds of primers of the present invention may be the following primer set including two specific primers.
(1') HLA-G gene amplification primer set:
   a primer set including (1a) a first primer comprising the nucleotide sequence of SEQ ID NO. 1 or the nucleotide sequence complementary thereto and (1b) a second primer comprising the nucleotide sequence of SEQ ID NO. 3 or the nucleotide sequence complementary thereto;
(2') HLA-E gene amplification primer set:
   a primer set including (2a) a first primer comprising the nucleotide sequence of SEQ ID NO. 5 or the nucleotide sequence complementary thereto and (2b) a second primer comprising the nucleotide sequence of SEQ ID NO. 7 or the nucleotide sequence complementary thereto; or
(3') HLA-F gene amplification primer set:
   a primer set including (3a) a first primer comprising the nucleotide sequence of SEQ ID NO. 9 or the nucleotide sequence complementary thereto and (3b) a second primer comprising the nucleotide sequence of SEQ ID NO. 11 or the nucleotide sequence complementary thereto.

In the above primer sets (1') to (3'), two specific primers are used. These two specific primers are set so as to generate an amplification product having a large size of 4500 bp or more (Examples). Therefore, the above primer sets (1') to (3') are preferably used in a gene amplification method capable of easily generating an amplification product having a large size. Examples of such a gene amplification method include PCR, STRAND Displacement Amplification (SDA), Hybrid Capture, Ligase Chain Reaction (LCR), and Cleavase Invader.

In a case where the one or more kinds of primers of the present invention are a primer set including the above two specific primers, it is possible to amplify an HLA allele of a target gene further highly comprehensively as compared with a case where the one or more kinds of primers of the present invention are a primer set including one specific primer, and it is also possible to amplify the entire length of the gene including an untranslated region of the gene.

A primer comprising SEQ ID NO. 1, 3, 5, 7, 9, or 11, or the nucleotide sequence complementary thereto may have an additional nucleotide added at a 5' end thereof or a 3' end thereof. Examples of such a nucleotide include a complementary nucleotide to a corresponding nucleotide at a target site. The number of complementary nucleotides is not particularly limited, and may be, for example, 1 to 10, and preferably 1 to 5. When such a nucleotide is added to the primer, annealing to the target site can be performed more stably, and detection or amplification efficiency can be improved. However, the primer comprising SEQ ID NO. 1, 3, 5, 7, 9, or 11, or the nucleotide sequence complementary thereto, itself can be annealed well to the target site, and therefore no additional nucleotide may be added to a 5' end thereof or a 3' end thereof. In addition, the primer may be chemically modified (for example, modified with an amino group), or may have a linker added.

In a certain embodiment, the first and second primers in each of which an additional nucleotide is added to a 5' end or a 3' end may be as follows.
(1a) The first primer comprising the nucleotide sequence of SEQ ID NO. 1 or the nucleotide sequence complementary thereto may be (1a') a first primer comprising the nucleotide sequence of SEQ ID NO. 2 or the nucleotide sequence complementary thereto.
(1b) The second primer comprising the nucleotide sequence of SEQ ID NO. 3 or the nucleotide sequence complementary thereto may be (1b') a second primer comprising the nucleotide sequence of SEQ ID NO. 4 or the nucleotide sequence complementary thereto.
(2a) The first primer comprising the nucleotide sequence of SEQ ID NO. 5 or the nucleotide sequence complementary thereto may be (2a') a first primer comprising the nucleotide sequence of SEQ ID NO. 6 or the nucleotide sequence complementary thereto.
(2b) The second primer comprising the nucleotide sequence of SEQ ID NO. 7 or the nucleotide sequence complementary thereto may be (2b') a second primer comprising the nucleotide sequence of SEQ ID NO. 8 or the nucleotide sequence complementary thereto.
(3a) The first primer comprising the nucleotide sequence of SEQ ID NO. 9 or the nucleotide sequence complementary thereto may be (3a') a first primer comprising the nucleotide sequence of SEQ ID NO. 10 or the nucleotide sequence complementary thereto.
(3b) The second primer comprising the nucleotide sequence of SEQ ID NO. 11 or the nucleotide sequence complementary thereto may be (3b') a second primer comprising the nucleotide sequence of SEQ ID NO. 12 or the nucleotide sequence complementary thereto.

An outline of the nucleotide sequences of SEQ ID NOs: 1 to 12 is as follows (for details, see Table 5).

**Table 1. Outline of the nucleotide sequences of SEQ ID NOs: 1 to 12**

| Target | type | Primer Sequence (5'→3') | SEQ ID NO |
|---|---|---|---|
| HLA-G | Forward | **t a g t g a g g g g c a t t g t** | 1 |
| | | g c a c **t a g t g a g g g g c a t t g t** | 2 |
| | Reverse | **c t c t c c t c t c g g g a a g t** | 3 |
| | | g a c **c t c t c c t c t c g g g a a g t** | 4 |
| HLA-E | Forward | **c g g c c t g g a g a a a t t c** | 5 |
| | | c **c g g c c t g g a g a a a t t c** a g t | 6 |
| | Reverse | **a g a a a a c a g t g c c a c g** | 7 |
| | | c c c a **a g a a a a c a g t g c c a c g** | 8 |
| HLA-F | Forward | **a a g a g t c c g g g g a g t** | 9 |
| | | c a a **a a g a g t c c g g g_g_a_g_ t** c c | 10 |
| | Reverse | **g c t c g t g a g g a a t g g** | 11 |
| | | a a a **g c t c g t g a g g a a t g g** g g | 12 |

A primer comprising SEQ ID NO. 2, 4, 6, 8, 10, or 12, or the nucleotide sequence complementary thereto may have an additional nucleotide(s) added at a 5' end thereof or a 3' end thereof. Examples of such a nucleotide include a complementary nucleotide to a nucleotide at a target site. The number of complementary nucleotides is not particularly limited, and may be, for example, 1 to 10, and preferably 1 to 5. When such a nucleotide is added to the primer, annealing to the target site can be performed more stably, and detection or amplification efficiency can be improved. However, a primer comprising SEQ ID NO. 2, 4, 6, 8, 10, or 12, or the nucleotide sequence complementary thereto, itself can be annealed very well to the target site, and therefore no additional nucleotide may be added to a 5' end thereof or a 3' end thereof. In addition, the primer may be chemically modified (for example, modified with an amino group), or may have a linker added.

In a primer comprising SEQ ID NOs. 1 to 12 or the nucleotide sequence complementary thereto, a functional moiety such as a labeling moiety that enables identification (for example, a non-complementary nucleotide-containing nucleotide sequence such as a barcode sequence) may be added to a 5' end thereof. Examples of such a functional moiety include Universal Sequence (PacBio) and Adapter Overhang Nucleotide Sequence (Illumina).

The primer as described above used in the present invention can be designed so as to have a specific nucleotide number (nucleotide length). Such a nucleotide number may be, for example, 15 or more, preferably 16 or more, more preferably 17 or more, still more preferably 18 or more, and particularly preferably 20 or more. Such a nucleotide number may be, for example, 50 or less, preferably 45 or less, more preferably 40 or less, still more preferably 35 or less, and particularly preferably 30 or less. More specifically, such a nucleotide number may be, for example, 15 to 50, preferably 16 to 45, more preferably 17 to 40, still more preferably 18 to 35, and particularly preferably 20 to 30.

The present invention also provides a method for detecting an HLA gene. The method of the present invention includes detecting one or more kinds of HLA genes selected from the group consisting of an HLA-G gene, an HLA-E gene, and an HLA-F gene in a sample obtained from a human subject using the one or more kinds of primers of the present invention.

As a sample obtained from a human subject, any sample containing a target of the one or more kinds of primers of the present invention can be used. Therefore, the sample obtained from a human subject is not particularly limited as long as the sample contains a genome and/or a transcript of a target HLA gene, and examples thereof include a sample collected from a human and a sample prepared from a sample collected from a human (for example, a cell sample). Preferably, a genome-containing sample is used as the sample. The genome-containing sample is preferably a minimally invasive sample. Examples of such a sample include hair, saliva, blood (for example, whole blood, plasma, or serum), and mucosa (for example, oral mucosa or nasal mucosa).

For detection of an HLA gene in a sample, genomic DNA or a transcript of an HLA gene may be extracted from the sample. Such extraction can be performed by any method. The extracted genomic DNA or transcript of an HLA gene can be subjected to an appropriate gene amplification method (for example, PCR or LAMP) depending on the intended size of an amplification product. Alternatively, the sample may be directly subjected to a gene amplification method (for example, direct PCR).

The method of the present invention is useful for, for example, transplantation, selection of a cancer therapeutic agent/therapeutic method (for example, a cancer vaccine), or HLA examination for determining a disease risk.

The present invention also provides an HLA gene detection reagent containing the one or more kinds of primers of the present invention. According to the reagent of the present invention, the method of the present invention can be easily performed.

The reagent of the present invention can contain the one or more kinds of primers of the present invention in a form of powder (for example, lyophilization) or a solution. The solution is preferably an aqueous solution. Examples of the aqueous solution include water (for example, sterile distilled water) and a buffer. Examples of the buffer include a TE (Tris-EDTA) buffer, a hydrochloric acid-potassium chloride buffer, a glycine-hydrochloric acid buffer, a citrate buffer, an acetate buffer, a citrate-phosphate buffer, a phosphate buffer, a Tris-hydrochloric acid buffer, a glycine-sodium hydroxide buffer, a carbonate-bicarbonate buffer, a borate buffer, and a tartrate buffer. When the reagent of the present invention is a solution containing the one or more kinds of primers of the present invention, the concentration of the primers in the solution varies depending on a factor such as use of the primers and a dilution ratio when the primers are used, but may be, for example, 0.1 to 100 mM, and preferably 1 to 10 mM. The solution may contain another component such as a stabilizer.

The invention also provides an HLA gene detection kit containing (a) the one or more kinds of primers of the invention and (b) a polymerase. According to the kit of the present invention, the method of the present invention can be easily performed.

As the polymerase, an appropriate polymerase according to the type of gene amplification method can be used. For example, in a case of PCR, a heat-resistant polymerase is preferably used, and in a case of LAMP, a strand displacement polymerase is preferably used. The polymerase is preferably a DNA polymerase.

The kit of the present invention may contain an additional component in addition to (a) and (b). Examples of such a component include a deoxynucleoside triphosphate (dNTP) mixture, a reaction buffer, a molecular weight marker, and a control (for example, reference standards of amplification products of various HLA alleles). When the kit of the present invention contains additional components, the components may be provided in a form in which the components are isolated from each other, for example, in a form in which the components are stored in different containers (for example, tubes), but may be provided in a form in which the components are mixed in advance (for example, PreMix) or the like.

In a certain embodiment, detection may be performed in real time (for example, real time PCR). In this case, examples of a method for enabling detection in real time include an intercalator method and a fluorescent substance-labeled probe method. Thus, when detection in real time is intended, the kit of the present invention may further contain a fluorescent substance or a fluorescent substance-labeled probe as an additional component. Examples of the fluorescent substance include a fluorescent substance (for example, SYBR (registered trademark) Green I) used in an intercalator method. Examples of the fluorescent substance-labeled probe include a probe in which a fluorescent substance is bound to one of a 5' end and a 3'end and a quencher is bound to the other of the 5' end and the 3' end (for example, a TaqMan (registered trademark) probe).

### EXAMPLES

The present invention will be described in detail with reference to the following Examples, but the present invention is not limited to the following Examples.

Primer design procedures and evaluation for performing gene-specific PCR on three HLA class Ib genes (HLA-E, HLA-F, and HLA-G) will be described.

### <Primer design>

One set of primers were designed for each of the genes (HLA-G_v1, HLA-E_v1, and HLA-F_v1) by executing PCR primer design tool Primer 3 on regions stored in a human genome reference sequence (genome assembly version hg 38) and Alternate sequences (chr6_GL000250v2_alt, chr6_GL000251v2_alt, chr6_GL000252v2_alt, chr6_GL000253v2_alt, chr6_GL000254v2_alt, chr6_GL000255v2_alt, chr6_GL000256v2_alt, and chr6_GL383533v1_alt).

### <Confirmation of HLA allele comprehensiveness>

It was confirmed whether the designed primer set could comprehensively PCR-amplify an HLA allele in silico using published whole genome sequence (WGS) data.

Published WGS data for 145 cases from HipSci Resource were downloaded from the European Nucleotide Archive (ENA, https://www.ebi.ac.uk/ena,study PRJEB 15299), and a FASTQ file including only a read that might be derived from an HLA gene and the vicinity thereof was created using BWA 0.7.17 and samtools 1.10.

HLA allele calling was performed using HLAHD 1.2.1 on the created FASTQ file of each sample (Tables 2, 3, and 4).

**Table 2. HLA-E alleles of 145 cases from HipSci Resource**

| # | HLA alleles | The number of alleles |
|---|---|---|
| 1 | E*01:01:01 | 175 |
| 2 | E*01:03:02 | 90 |
| 3 | E*01:06 | 8 |
| 4 | E*01:03:01 | 7 |
| 5 | E*01:03:05 | 6 |
| 6 | E*01:09 | 2 |
| 7 | E*01:03:06 | 1 |
| 8 | E*01:05 | 1 |

**Table 3. HLA-F alleles of 145 cases from HipSci Resource**

| # | HLA alleles | The number of alleles |
|---|---|---|
| 1 | F*01:01:01 | 193 |
| 2 | F*01:03:01 | 50 |
| 3 | F*01:01:02 | 39 |
| 4 | F*01:02 | 5 |
| 5 | F*01:04:01 | 2 |
| 6 | F*01:01:04 | 1 |

**Table 4. HLA-G alleles of 145 cases from HipSci Resource**

| # | HLA alleles | The number of alleles |
|---|---|---|
| 1 | G*01:01:01 | 125 |
| 2 | G*01:01:02 | 64 |
| 3 | G*01:04:01 | 27 |
| 4 | G*01:01:03 | 20 |
| 5 | G*01:06 | 20 |
| 6 | G*01:03:01 | 9 |
| 7 | G*01:01:22 | 8 |
| 8 | G*01:05N | 5 |
| 9 | G*01:04:04 | 4 |
| 10 | G*01:01:08 | 2 |
| 11 | G*01:01:20 | 2 |
| 12 | G*01:01:06 | 1 |
| 13 | G*01:01:17 | 1 |
| 14 | G*01:01:23 | 1 |
| 15 | G*01:04:03 | 1 |

For the created FASTQ file of each sample, a consensus sequence of an HLA class Ib gene and a sequence in the vicinity thereof was created using freebayes 1.3.1, whatshap 1.0, and bcftools 1.10.2, and correlated with HLA allele information of each sample.

It was evaluated whether a binding site to be a core sequence of the designed primer was present in the consensus sequence of each of the 145 cases from HipSci Resource using Bowtie 2 2.3.5.1, and it was confirmed that the binding site was present in the consensus sequence.

### <Comparison of HLA allele comprehensiveness in HLA-G gene primer>

For known HLA-G gene primers (Non Patent Literature 2), comprehensiveness to the consensus sequence of each of the 145 cases from HipSci Resource was evaluated using Bowtie 2 2.3.5.1. As a result, among 145 cases (the number of alleles was 290), there was a mismatch in 14 alleles, suggesting that there is a high possibility that PCR amplification is not observed or uniform amplification is affected.

Meanwhile, in both a forward primer and a reverse primer of the designed primer set HLA-G_v1, there was no mismatch in all 290 alleles, and therefore it was confirmed that allele comprehensiveness was very high.

### <Confirmation of specific PCR amplification by experiment>

In order to experimentally confirm whether the primer sets presented in Table 5 could specifically perform PCR amplification, a PCR reaction was performed using each HLA class Ib gene-specific primer set with genomic DNA extracted from a cell line (Daudi: D) established from an African population, a cell line (AKIBA: A) established from a Japanese population, or human peripheral blood mononuclear cells (L7) derived from Hispanic Americans as a template. Nuclease-free Water (N) was used as a non-template control.

**Table 5. Primer set particularly excellent in amplification specificity of HLA class Ib genes**

| HLA gene | Name of set | Name of primer | Primer sequence* (5'→3') | SEQ ID NO |
|---|---|---|---|---|
| HLA-G | HLA-G_v1 | HHA-G_v1_Fw | gcac**tagtgaggggcattgt** | 2 |
| | | HLA-G_v1_Rv | gacctctcctctc**gggaagt** | 4 |
| HLA-E | HLA-E_v1 | HLA-E_v1_Fw | c**cgg**cct**gg**a**gaaattc**agt | 6 |
| | | HLA-E_v1_Rv | ccca**agaaaacagtgccacg** | 8 |
| HLA-F | HLA-F_v1 | HLA-F_v1_Fw | caa**aagagtccggggagt**cc | 10 |
| | | HLA-F_v1_Rv | aaa**gctcgtgaggaatgg**gg | 12 |

| | | | | |
|---|---|---|---|---|
| Underlined bold portion indicates core sequence. For details, see identification of core sequence> described later. | | | | |

In the PCR reaction, a 25 µL solution in total including 12.5 µL of PrimeSTAR GXL Premix (2X), 2.5 µL of a forward primer (2 µM), 2.5 µL of a reverse primer (2 µM), 5 µL of Nuclease-free Water, and 2.5 µL of a genomic DNA solution (5 ng) was adjusted using PrimeSTAR GXL Premix (Takara Bio Inc.). This solution was kept at 94°C for one minute. Subsequently, a process including two steps consisting of a step of keeping the solution at 98°C for 10 seconds and a step of keeping the solution at 68°C for eight minutes was repeatedly performed 30 times. Note that for this PCR, Veriti 96 Well Thermal Cycler (Thermo Fisher Scientific) was used.

Next, after the PCR reaction, the PCR amplification product was purified using Sera-Mag Select (Cytiva), and eluted with 25 µL of Buffer EB (QIAGEN).

The PCR amplification product after purification was subjected to electrophoresis using Genomic DNA ScreenTape system (Agilent Technologies, Inc.), and a status of PCR amplification was confirmed (FIG. 1).

### <Sequencing using long read sequencer>

The PCR amplification product was sequenced using a Sequel system (Pacific Biosciences of California, Inc.).

PCR amplification was performed using the primer sets presented in Table 5 and using SMRTbell Express Template Prep Kit 2.0 in such a manner that setting of a measuring instrument, reaction conditions, and an addition amount of a reagent were basically in accordance with Procedure & Checklist Part Number 101-791-700 version 02 provided by PacBio Inc.

For the consensus sequence obtained by the Sequel system, HLA allele typing was performed using IMGT/HLA database as a reference sequence.

Analysis was performed using, as a template, genomic DNA extracted from a cell line (Daudi: D) established from an African population, a cell line (AKIBA: A) established from a Japanese population, and six cases of human peripheral blood mononuclear cells derived from Hispanic Americans (L7, L222, L255, L275, L340, and L365).

As a result, it was confirmed that the primer sets narrowed down this time could highly comprehensively amplify a large number of HLA allele groups including a putative novel HLA allele (Table 6).

**Table 6. Allele typing by long read sequencing of HLA class Ib gene**

| Sample | HLA-E | HLA-F | HLA-G |
|---|---|---|---|
| D | E*01:03:02:01 | F*01:01:01:09 | G*01:01:02 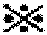 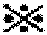 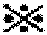 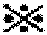 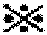 |
| | E*01:03:05 | F*01:01:01 | G*01:01:0.2:01 |
| A | E*01:03:01:01 | F*01:01:02:10 | G*01:04:01:02 |
| L7 | E*01:03:02:01 | F*01:01:01:08 | G*01:01:01:01 |
| | E*01:01:01:03 | F*01:01:01:01 | G*01:01:01:04 |
| L222 | E*01:06 | F*01:03:01:04 | G*01:01:01:05 |
| | E*01:01:01:01 | F*01:01:01:11 | G*01:03:01:02 |
| 1255 | E*01:03:02:01 | F*01:01:01 | G*01:01:01:01 |
| | E*01:03:01:01 | F*01:01:01:17 | G*01:01:03 |
| L275 | E*01:03:02:01 | F*01:01:02 | G*01:01:01:01 |
| | E*01:01:01:06 | F*01:01:01:17 | G*01:04:01:01 |
| L340 | E*01:01:01:01 | F*01:01:01:08 | G*01:01:02:01 |
| | E*01:03:02:01 | F*01:01:01:01 | G*01:01:01:01 |
| L365 | E*01:01:01:01 | F*01:01:01:18 | G*01:04:01:01 |
| | E*01:03:05 | F*01:01:01:09 | G*01:01:01:05 |

| | | | |
|---|---|---|---|
| 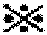 indicates a sequence which deduced as novel allele since there is no s equence completely corresponding to it in IMGT/HLA database. | | | |

### <Identification of core sequence>

In order to specify a core sequence of a forward primer or a reverse primer, the length of the designed primer was shortened by one nucleotide from a 5' side of the designed primer and a 3' side thereof. It was confirmed whether the sequence completely matched the human genome reference sequence (genome assembly version hg 38), and a core sequence that uniquely matched an upstream and a downstream of a target gene was identified (the sequence of the underlined bold portion in Table 5). It was confirmed that the binding site of this core sequence uniquely and completely matched the consensus sequence of the target gene of each of primers in the 145 cases from HipSci Resource.

## Claims

1. One or more kinds of HLA gene primers selected from the group consisting of the following (1) to (3):
(1) one or more kinds of HLA-G gene primers which are
(1a) a first primer comprising the nucleotide sequence of SEQ ID NO. 1 or the nucleotide sequence complementary thereto and/or (1b) a second primer comprising the nucleotide sequence of SEQ ID NO. 3 or the nucleotide sequence complementary thereto;
(2) one or more kinds of HLA-E gene primers which are
(2a) a first primer comprising the nucleotide sequence of SEQ ID NO. 5 or the nucleotide sequence complementary thereto and/or (2b) a second primer comprising the nucleotide sequence of SEQ ID NO. 7 or the nucleotide sequence complementary thereto; and
(3) one or more kinds of HLA-F gene primers which are
(3a) a first primer comprising the nucleotide sequence of SEQ ID NO. 9 or the nucleotide sequence complementary thereto and/or (3b) a second primer comprising the nucleotide sequence of SEQ ID NO. 11 or the nucleotide sequence complementary thereto.

2. The one or more kinds of HLA gene primers according to claim 1, wherein the one or more kinds of HLA gene primers are HLA gene amplification primer sets selected from the group consisting of the following (1') to (3'):
(1') an HLA-G gene amplification primer set including
(1a) a first primer comprising the nucleotide sequence of SEQ ID NO. 1 or the nucleotide sequence complementary thereto and (1b) a second primer comprising the nucleotide sequence of SEQ ID NO. 3 or the nucleotide sequence complementary thereto;
(2') an HLA-E gene amplification primer set including
(2a) a first primer comprising the nucleotide sequence of SEQ ID NO. 5 or the nucleotide sequence complementary thereto and (2b) a second primer comprising the nucleotide sequence of SEQ ID NO. 7 or the nucleotide sequence complementary thereto; and
(3') an HLA-F gene amplification primer set including
(3a) a first primer comprising the nucleotide sequence of SEQ ID NO. 9 or the nucleotide sequence complementary thereto and (3b) a second primer comprising the nucleotide sequence of SEQ ID NO. 11 or the nucleotide sequence complementary thereto.

3. The one or more kinds of HLA gene primers according to claim 1 or 2, wherein
(1a) the first primer comprising the nucleotide sequence of SEQ ID NO. 1 or the nucleotide sequence complementary thereto is (la') a first primer comprising the nucleotide sequence of SEQ ID NO. 2 or the nucleotide sequence complementary thereto,
(1b) the second primer comprising the nucleotide sequence of SEQ ID NO. 3 or the nucleotide sequence complementary thereto is (1b') a second primer comprising the nucleotide sequence of SEQ ID NO. 4 or the nucleotide sequence complementary thereto,
(2a) the first primer comprising the nucleotide sequence of SEQ ID NO. 5 or the nucleotide sequence complementary thereto is (2a') a first primer comprising the nucleotide sequence of SEQ ID NO. 6 or the nucleotide sequence complementary thereto,
(2b) the second primer comprising the nucleotide sequence of SEQ ID NO. 7 or the nucleotide sequence complementary thereto is (2b') a second primer comprising the nucleotide sequence of SEQ ID NO. 8 or the nucleotide sequence complementary thereto,
(3a) the first primer comprising the nucleotide sequence of SEQ ID NO. 9 or the nucleotide sequence complementary thereto is (3a') a first primer comprising the nucleotide sequence of SEQ ID NO. 10 or the nucleotide sequence complementary thereto, and
(3b) the second primer comprising the nucleotide sequence of SEQ ID NO. 11 or the nucleotide sequence complementary thereto is (3b') a second primer comprising the nucleotide sequence of SEQ ID NO. 12 or the nucleotide sequence complementary thereto.

4. A method for detecting an HLA gene, comprising:
detecting one or more kinds of HLA genes in a sample obtained from a human subject using the one or more kinds of HLA gene primers according to any one of claims 1 to 3,
wherein the one or more kinds of HLA genes are selected from the group consisting of an HLA-G gene, an HLA-E gene, and an HLA-F gene.

5. The method according to claim 4, wherein the one or more kinds of HLA genes are detected by amplifying the one or more kinds of HLA genes.

6. An HLA gene detection reagent comprising the one or more kinds of HLA gene primers according to any one of claims 1 to 3.

7. An HLA gene detection kit comprising:
(a) the one or more kinds of HLA gene primers according to any one of claims 1 to 3; and
(b) a polymerase.
